# EUROPEAN PATENT SPECIFICATION

(11) **EP 1 599 158 B1**
(45) Date of publication and mention of the grant of the patent: **04.07.2007**
(21) Application number: 04701344.6
(22) Date of filing: 12.01.2004
(51) Int. Cl.: A61F 5/01

(54) **ANKLE BRACE**
FUSSGELENKSCHIENE
ATTELLE POUR CHEVILLE

(30) Priority: 06.03.2003 DK 200300346
(43) Date of publication of application: 30.11.2005
(73) Proprietor: Hjorth, Knud, 4621 Gadstrup (DK)
(72) Inventor: Hjorth, Knud, 4621 Gadstrup (DK)
(86) International application number: PCT/DK2004/000010
(87) International publication number: WO 2004/078076

(56) References cited:
- DE-A- 19 941 368
- DE-C- 308 526
- US-A- 5 860 423

## Description

The invention relates to an ankle brace - Orthosis - which can be used in all cases, where nerves, muscles and similar in the legs or elsewhere in the nervous system are damaged in such a way, that the person will not be able to raise the front part of the foot sufficient to have a normal walk, the so called drop foot.

The ankle brace is constructed so, that the sole to be put into the shoe, forms the base of the ankle brace.

On the sole is mounted a heel counter by means of a click arrangement. On the heel counter is mounted a joint, which is constructed thus, that the sole and the heel counter freely are able to move in all directions compared with the upper part of the ankle brace and this means, that the foot has free movement in the ankle joint in all directions and so the person will achieve a free and natural walk and the crossed straps cause, that the foot is placed in a normal position after every step in contrast to all other known ankle braces, where the movement of the foot is fixed to a movement back and forth, but not sideways. The movement of the ankle joint in all directions causes furthermore, that the steel bar and the double counter are able to rock completely to the rear by means of which, there is no form of hindrance, when the foot is put into the shoe.

The assembling of the heel counter and the sole by means of a click arrangement causes, that the different sizes of sole and counter as a matter of course can be clicked together and be adjusted to the individual shoe and this in connection with, that the upper double counter is adjustable and that the length of the straps by moving the hole in the strap, which is hitched on the double counter, can be adjusted individually, causes, that the ankle brace immediately can be adjusted to the shoe of the individual person.

With the double counter an improvement has been achieved, that where other counters glide up and down direct upon the calf of the leg, the movement between the inner and the outer counter will cause, that the movement up and down against the calf will be caught by the movement of the joint.

We can refer to the following well-known ankle braces:
DE 297 04 669 U1
DE 199 41 368 A1
both having an ankle brace with a bar on each side of the foot and fastened thus, that the sideway movements are inflexible, and
US 5 860 423
in which the ankle brace on the heel counter is fastened in 2 points by which the sideway movements are not possible.

As regards DE 199 41 368 A1 it is stated, that the upper counter can be taken to pieces by which it will be possible to force the ankle braces from each other in order to make it easier to put them on.

Document DE 308526 describes an orthosis comprising an upper part and a lower part joined through a joint allowing sideways movement additionally to backwards and forwards movement of the lower part of the orthosis compared with the upper part.

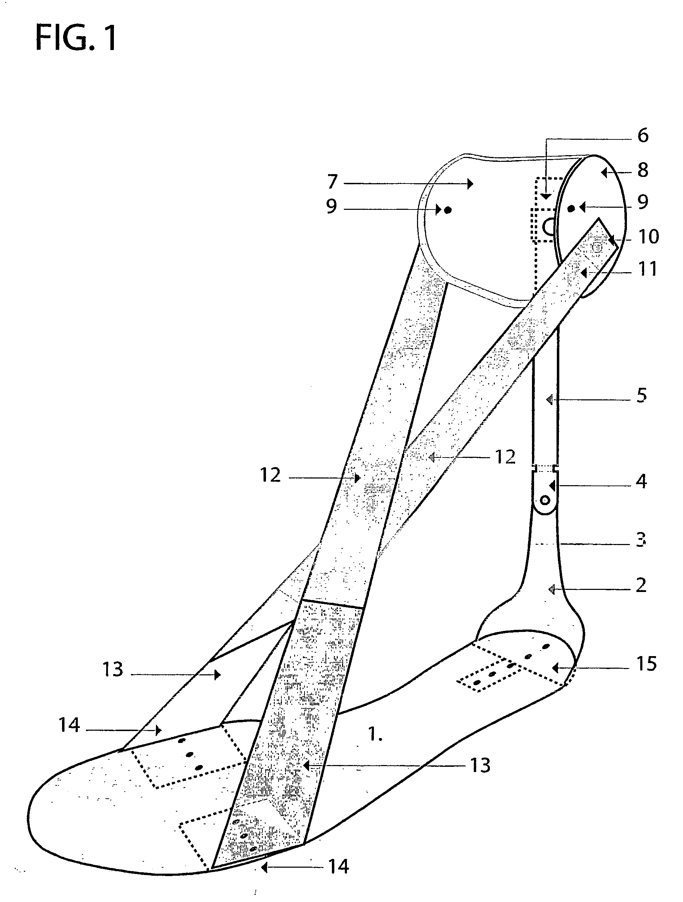

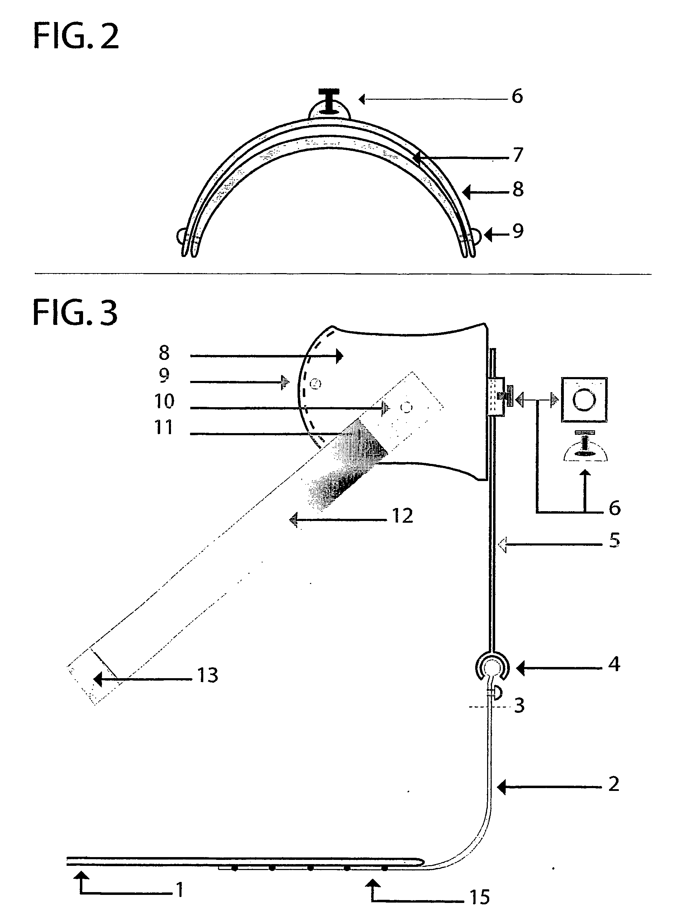

The invention will be explained more precisely in the following, referring to the drawings fig. 1 showing the ankle brace from in front, fig. 2 showing the double counter and fittings and fig. 3 showing the ankle brace from the side.

The ankle brace consists of a sole (1), which will be fitted to the customers footwear. The sole will be provided with a vertical heel counter (2), which will be fitted to the heel counter of the shoe. This heel counter ends about 1 centimetre above the heel counter of the shoe, marked by a line (3).

The joining of the sole and the vertical heel counter is made by a click system, that means that on the underside of the sole, small downward turned knobs are cast. In the heel counter going under the sole (15) are made holes, which diameter and intervals are as the knobs thus, that the sole and the heel counter can be clicked together. The heel counter ends with a joint (4), which allows movements backwards and forwards and at the same time to both sides, which means full movement.

In vertical continuation of this joint is mounted a steel bar (5)

On the steel bar is mounted a sliding fitting which can be moved up and down the steel bar and can be secured in the height wanted by means of a stretching screw (6).

On this gliding fitting is mounted a semicircular forward turned counter (8) thus, that the centre of the circle is mounted on the sliding fitting and so, that the ends of the counter go equally forward on both sides of the leg. This counter will because of the direct connection with the sole in the shoe (2, 4, 5, 6, 15) follow the movements of the shoe heel while walking.

In order to counteract these movements, the outer counter (8) is provided with an inner counter (7), which lies upon the calf of the leg.

The inner counter is fastened to the outer counter in both sides with a moveable joint (9) by which the up and down going movements are counteracted.

On the outer side of the outer counter are in both sides mounted a hook arrangement (10) or a piece of Welco-belt for fastening in both sides a 2-3 centimetre wide elastic strap (11-14), which is composed of the pieces 11-13, of which the piece 11 is an inelastic strap. After that follows the piece 12 which is an elastic strap and there after follows the piece 13 which is an inelastic strap. The latter is mounted under the sole at 14 by use of a click arrangement like that, used under the sole and the heel counter (15).

In the last-mentioned case knobs will be cast under the sole in both sides, so that the holes in the bottom of the strap (14) fit the knobs in both sides under the sole.

By this one achieves to be able to use the single strap on both sides of the foot, just by an inversion of the strap.

The piece of the strap 13 is placed inside the shoe, while the piece of the strap 12 comes out of the shoe and lies freely and turns half round over the instep and goes up to the piece of the strap 11 which is fastened at 10.

## Claims

1. Ankle brace to be put on a foot, for a person not able to sufficiently raise the front-part of the foot during walking, wherein the ankle brace consists of a lower part, said lower part being a sole (1) fastened with a click system (15) to a heel counter (2), and an upper part, said upper part being a bar (5) with a fastened adjustable fitting (6) with an adjustable double counter (7, 8) fastened to said fitting (6), wherein said upper and lower part in use are put together in front by two elastic adjustable straps (11-13), which crosswise over the instep go from a hook arrangement (10) on the double counter (7, 8) to the sole (1), wherein said straps (11-13) are fastened under the sole (1) with a click system and said upper and lower part of the brace are, in use, put together on the backside of the leg, at the calf, in a moveable joint (4),
**characterised in that** said joint (4) is constructed such, that it allows free movement of the lower part compared with the upper part of the ankle brace backwards and forwards and sideways, so that the foot has free movement in the ankle joint in all directions.

2. Ankle brace according to claim 1, **characterised in that** the double counter (7, 8) is a semicircular forward turned counter with two ends, said double counter comprising an inner counter (7) and an outer counter (8), wherein the ends of the double counter, in use, go equally forward on both sides of the leg and wherein the inner counter (7) is fastened to the outer counter (8) at both ends with a moveable joint (9).

## Patentansprüche

1. Fußgelenkschiene, die am Fuß einer Person angebracht wird, die nicht in der Lage ist, den Vorderteil des Fußes beim Gehen ausreichend anzuheben, wobei die Fußgelenkschiene aus einem Unterteil besteht, das eine Sohle (1) ist, die mit einem Schnappverschluss (15) an einem Fersen-Gegengewicht (2) befestigt wird, und einem Oberteil, welches eine Stange (5) mit einem daran befestigten verstellbaren Fitting (6) mit einem verstellbaren doppelten Gegengewicht (7, 8) ist, das am genannten Fitting (6) befestigt ist, wobei das genannte Ober- und Unterteil im Gebrauch vorn durch zwei elastische, verstellbare Bänder (11-13) aneinander befestigt werden, welche über Kreuz über den Spann gehen von einer Haken-Vorrichtung (10) aus an dem doppelten Gegengewicht (7, 8) zur Sohle (1), wobei die genannten Bänder (11-13) mit einem Schnappverschluss unter der Sohle (1) befestigt werden, und der genannte Ober- und Unterteil der Schiene im Gebrauch an der Rückseite des Beines an der Wade in einem beweglichen Gelenk (4) zusammen geführt werden, das **dadurch gekennzeichnet ist, dass** das genannte Gelenk (4) so aufgebaut ist, dass es die freie Vorwärts- und Rückwärts- sowie seitliche Bewegung des Unterteils gegenüber dem Oberteil der Fußgelenkschiene gestattet, so dass der Fuß sich im Fußgelenk in allen Richtungen frei bewegen kann.

2. Fußgelenkschiene nach Anspruch 1, **dadurch gekennzeichnet, dass** das doppelte Gegengewicht (7, 8) ein halbkreisförmiges, vorwärts gerichtetes Gegengewicht mit zwei Enden ist, wobei das genannte doppelte Gegengewicht
aus einem inneren (7) und einem äußeren Gegengewicht (8) besteht, wobei die Enden des doppelten Gegengewichtes im Gebrauch auf beiden Seiten des Beines gleichermaßen nach vorn gehen, und wobei das innere Gegengewicht (7) an beiden Enden mit einem beweglichen Gelenk (9) am äußeren Gegengewicht (8) befestigt ist.

## Revendications

1. Attelle pour cheville à poser sur le pied d'une personne qui n'est pas capable de soulever suffisamment la partie avant du pied durant la marche, ladite attelle se composant d'une partie inférieure, qui est une semelle (1) fixée à un contrefort (2) à l'aide d'un système à encliquetage (15), et d'une partie supérieure, qui est une barre (5) dotée d'une attache réglable fixe (6) à double contrefort réglable (7, 8) fixé à ladite attache (6). Dans cette attelle, lesdites parties inférieure et supérieure sont, lors de l'utilisation, assemblées à l'avant par deux sangles élastiques ajustables (11-13), qui vont d'un dispositif à crochet (10) sur le double contrefort (7, 8) à la semelle (1) en passant en croix sur le cou-de-pied. Dans cette attelle, lesdites sangles (11-13) sont fixées sous la semelle (1) à l'aide d'un système à encliquetage et lesdites parties supérieure et inférieure sont, lors de l'utilisation, assemblées à l'arrière de la jambe, à hauteur du mollet, dans une articulation mobile (4), qui se **caractérise en ce que** ladite articulation (4) est conçue de telle sorte qu'elle permet le libre mouvement de la partie inférieure de l'attelle pour cheville, par rapport à sa partie supérieure, vers l'arrière, vers l'avant et vers les côtés, si bien que le pied peut bouger librement dans tous les sens à hauteur de l'articulation tibio-tarsienne.

2. Attelle pour cheville selon la revendication 1, qui se **caractérise en ce que** le double contrefort (7, 8) est un contrefort demi-circulaire orienté vers l'avant et doté de deux extrémités, ledit double contrefort se composant d'un contrefort intérieur (7) et d'un contrefort extérieur (8). Dans cette attelle, les extrémités du double contrefort avancent, lors de l'utilisation, à parts égales sur les deux côtés de la jambe, et le contrefort intérieur (7) est fixé sur le contrefort extérieur (8) à hauteur des deux extrémités à l'aide d'une articulation mobile (9).
